# EUROPÄISCHE PATENTSCHRIFT

(11) **EP 0 961 602 B1**
(45) Veröffentlichungstag und Bekanntmachung des Hinweises auf die Patenterteilung: **06.03.2002**
(21) Anmeldenummer: 97950206.9
(22) Anmeldetag: 20.11.1997
(51) Int. Cl.: A61F 13/02, A61L 15/44

(54) **KONTUR EINES TRANSDERMALEN THERAPEUTISCHEN SYSTEMS**
CONTOUR OF A TRANSDERMAL THERAPEUTIC SYSTEM
CONTOUR DE SYSTEME THERAPEUTIQUE TRANSDERMIQUE

(30) Priorität: 16.12.1996 DE 19652269
(43) Veröffentlichungstag der Anmeldung: 08.12.1999
(73) Patentinhaber: LTS Lohmann Therapie-Systeme AG, 56626 Andernach (DE)
(72) Erfinder: Bracht, Stefan, D-56299 Ochtendung (DE)
(74) Vertreter: Flaccus, Rolf-Dieter, Dr.
(86) Internationale Anmeldenummer: EP9706485
(87) Internationale Veröffentlichungsnummer: WO9826740

(56) Entgegenhaltungen:
- WO-A-95/23687
- DE-A- 1 957 851
- DE-A- 19 511 976
- US-A- 4 666 441
- US-A- 4 719 909

## Beschreibung

Die Erfindung betrifft ein transdermales therapeutisches System (TTS) zur Abgabe von Wirkstoff an oder durch die Haut, mit einer auf der Haut vergleichsweise dauerhaft haftklebenden Schicht, deren Ränder von einer Kontur bestimmt sind. Unter TTS sind im folgenden sowohl teilflächig als auch vollflächig auf der Haut klebende transdermale therapeutische Systeme zu verstehen.

Ein technisches Problem bei der Entwicklung von TTS ist die Sicherstellung eines konturschlüssigen Verbundes mit der Haut beim Tragen für Zeiträume von 24 Stunden bis hin zu 7 Tagen oder mehr. Konturschlüssig heißt in diesem Zusammenhang, daß eine Kantenablösung des TTS von der Haut weitgehend vermieden wird.
Um einen haftklebenden Verbund eines TTS mit der Haut für vergleichsweise lange Zeiträume sicherzustellen, ist einerseits eine Optimierung der hautseitigen Kleberschicht erforderlich. Je nach Aufbau des TTS kann diese Schicht wirkstoffhaltig sein und außerdem pharmazeutische Hilfsstoffe enthalten, die die Hautpermeation des Wirkstoffs begünstigen. In solchen Fällen wird das Klebeverhalten der Schicht durch Art und Menge der enthaltenen Wirk- bzw. Hilfsstoffe mehr oder weniger stark beeinflußt.
Zusätzliche Einflüsse auf das Trageverhalten gehen andererseits von der Größe des TTS und vom Applikationsort am menschlichen Körper aus. Es resultieren aus diesen beiden Parametern maßgeblich die auf das TTS einwirkenden mechanischen Kräfte (z.B. Streck-, Stauch- und Scherkräfte), die zu einer zumindest partiellen Ablösung von der Haut führen können.

Die Einstellung optimaler Klebeeigenschaften ist eine die Formulierung der fakultativ wirkstoffhaltigen Kleberschicht betreffende und stark vom inneren und äußeren Aufbau des TTS abhängige Aufgabe. Die damit verbundenen Variabilitäten machen die Verallgemeinerung erfolgreicher Formulierungen weitgehend unmöglich. Für jedes neue TTS ist daher in der Regel eine mehr oder weniger aufwendige Optimierung der Klebeeigenschaften erforderlich.

Dagegen wird eine konturschlüssige Langzeitapplikation eines TTS auf der Haut in erheblichem Maße von den äußeren Formen eines TTS im Sinne des Verlaufs seiner Kontur beeinflußt, der je nach seiner Ausbildung die Ablösung der Kanten eines TTS beim Tragen auf der Haut mechanisch hindern und damit vorteilhaft verzögern können.

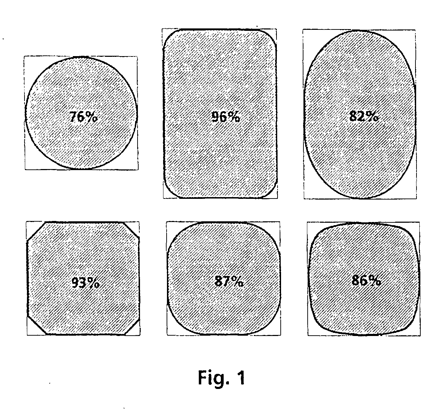

Die Festlegung der äußeren Kontur von TTS richtet sich gemäß dem Stand der Technik nach folgenden Kriterien:
1. Die Herstellung eines TTS in seiner endgültigen Form erfolgt üblicherweise durch Austrennen aus einem dünnnschichtigen, bahnförmigen Material. Es kann sich z.B. um ein getaktetes Stanzen mit flächigem Stanzwerkzeug oder um ein kontinuierliches Schneiden mit einem walzenförmigen Schneidwerkzeug handeln.
   Dabei zielt die Konturgebung eines TTS bevorzugt auf eine möglichst geringe Menge Abfall beim Stanzen oder Schneiden ab. Dies gilt insbesondere bei wirkstoffhaltigem Abfall. Da das Ausgangsmaterial in der Regel einen mehrschichtigen, komplexen Aufbau besitzt, ist eine Wiederverwertung der Abfälle häufig unmöglich oder unrentabel.
   Die Abfallreduzierung besitzt daher einen großen Stellenwert unter den Kriterien der Konturgebung bei der TTS-Herstellung.
2. Unter dem Aspekt verringerten Ablöseverhaltens eines aufgeklebten TTS auf der Haut werden spitze Winkel < 90° in der Kontur vermieden. Solche Spitzen stellen besonders exponierte Punkte für die Ablösung von der Haut dar und werden infolgedessen vermieden. Rechtwinklige Ecken werden abgerundet,was sich in der Praxis als geeignetes Mittel zur Reduzierung der Eckenablösung erwiesen hat und allgemeine Anwendung findet.
3. Die äußere Form eines TTS darf bei Patienten keinen Widerwillen gegen das Anbringen und Tragen auf der Haut hervorrufen. Sie muß daher eine einfache Anbringung und eine leichte Wiederentfernung begünstigen.
   Bewährt haben sich einfache geometrische Konturen. Dies hat im Stand der Technik zu TTS-Konturen geführt, die von den Formen des Kreises, der Ellipse, des Rechteckes, des Quadrates oder anderer Polygone bestimmt sind. Abb.1 zeigt eine beispielhafte Auswahl der üblichen Formen.

Das Dokument US 4,666,441 offenbart ein TTS, dessen Konturlinie konkave Abschnitte enthält. Es handelt sich um das Produkt "Estracombi" ® der Firma Ciba. Die Form dieses außerhalb der Fachsprache auch als "Brillenpflaster" bezeichneten Systems hängt jedoch erfindungsgemäß nicht mit einer Verbesserung des konturschlüssigen Verbundes mit der Haut zusammen. Vielmehr dient der Aufbau einer Kammerung des enthaltenen Flüssigkeitsreservoirs. Der Anteil der konkaven Konturlinienabschnitte an der Gesamtlänge der äußeren TTS-Kontur beträgt außerdem weniger als 10 Prozent.

Außerhalb der Gruppe von TTS sind andere hauthaftende Systeme bekannt, deren Konturlinien teilweise konkave Abschnitte beinhalten. Es handelt sich dabei im Unterschied zu TTS um wesentlich flexiblere Pflaster oder pflasterähnliche Elemente wie:
- Wundpflaster zur Anwendung am Auge(z.B. US 4,709,695).
- UV-Schutzpflaster zur Anwendung im Bereich unterhalb des Auges (z.B. US 4,719,909).
- Hauthaftende Hydrogele zur kosmetische Beeinflussung des Hautzustandes im Bereich unterhalb des Auges (z.B. DE 44 46 380).
- Pflaster, um das Einwachsen des Fußnagels an der Großzehe zu verhindern ( z.B. DE 38 23 889).
- Pflaster, um die Nasenflügel zu spreizen und dadurch die Nasenatmung zu verbessern (z.B. Breath Wright ™ in USA).

Allen diesen relativ flexiblen hauthaftenden Systemen ist gemeinsam, daß bei ihnen der Verlauf von konkaven Kontur durch individuelle anatomische Gegebenheiten am Applikationsort vorgegeben wird. Die Formgebung ist dabei der menschlichen Anatomie angepaßt.

Der Erfindung liegt die Aufgabe zugrunde, bei einem TTS Konturen zu verwirklichen, die derart ausgebildet sind, daß sie einen wesentlichen Beitrag zur Verbesserung und Verlängerung eines konturschlüssigen Verbundes des TTS mit der Haut ergeben.

Die Lösung der Aufgabe gelingt bei einem TTS der im Oberbegriff von Anspruch 1 genannten Art mit der Erfindung dadurch, daß in deren Kontur wenigstens zwei konkave Abschnitte vorhanden.
Weitere vorteilhafte Ausgestaltungen sind entsprechend den Merkmalen der Unteransprüche vorgesehen.

Dieser Lösungsweg ist überraschenderweise trotz etwa 2 Jahrzehnten der TTS-Entwicklung und einer Vielzahl von Produkten auf dem Weltmarkt bisher nicht beschritten worden. Die Entwicklung von TTS umfaßt bis heute als große Schwerpunkte die Optimierung der Wirkstoffabgabe und die Klebeeigenschaften der hauthaftenden Formulierung. Dem Einfluß der äußeren Gestalt insbesondere auf das Langzeittrageverhalten wurde dabei bisher wenig Beachtung geschenkt.

Bei den zwischen Hautarealen und einem selbstklebenden TTS wirkenden mechanischen Kräften handelt es sich überwiegend um Scherkräfte. Die dem TTS unterliegende Haut wird durch Streckung oder Beugung der jeweils nächstliegenden Gelenke mehr oder weniger stark gedehnt oder gestaucht. Ebenfalls zu Dehnung oder Stauchung eines solchen Hautareals führen abweichende Kontraktionen und Erschlaffungen der unterliegenden Skelettmuskulatur, die in fast allen Applikationsgebieten des menschlichen Körpers vorhanden ist.
Neben den Scherkräften, die von der beklebten Haut übertragen werden, wirken auf ein TTS weitere Scher- und Schälkräfte ein, sofern darüber Kleidung getragen wird.
Die Kräfte sind stark von der Beschaffenheit der äußeren Schicht des TTS sowie der überlagernden Textilien abhängig. Großen Einfluß auf die Ablösung des TTS von der Haut haben diese Schälkräfte insbesondere dann, wenn sich bereits ein kleiner Teil des TTS-Randes von der Haut gelöst hat. Diese in der Regel klebrigen Abhebungen werden von Textilien leicht mitgerissen und leiten so eine beschleunigte Abschälung des gesamten TTS ein.
Ein wichtiger Sonderfall der Wechselwirkung des TTS mit Textilien tritt während der Schlafphase des Patienten ein. An fast jedem der vorgenannten üblichen Applikationsort besteht die Möglichkeit, daß die schlafende Person auf das TTS selbst zu liegen kommt. Dies führt bei Körperbewegungen leicht zu einer großflächigen Übertragung erheblicher Scher- bzw. Schälkräfte auf das TTS.

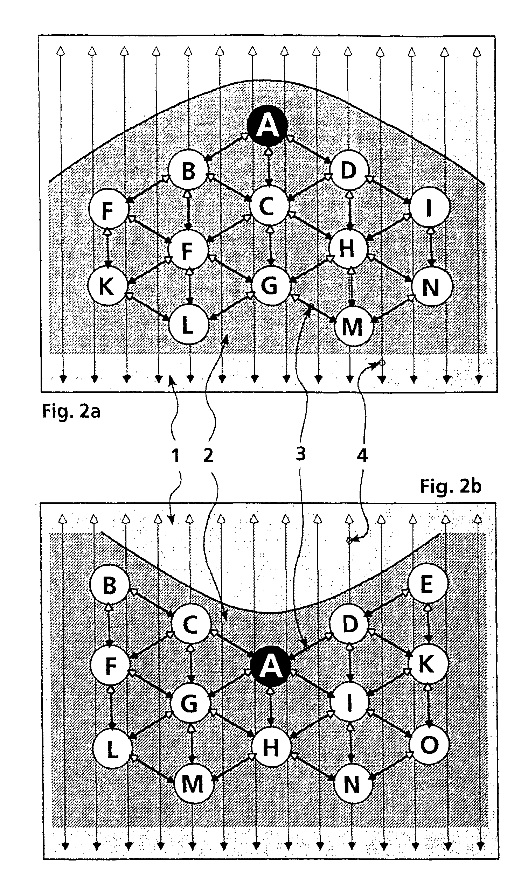

Die Auswirkung von Scherkräften auf die Haftklebung eines TTS wird im folgenden modellhaft vereinfacht dargestellt. In der Fläche eines auf die Haut applizierten TTS lassen sich hypothetische Haft- bzw. Klebepunkte darstellen. Diese nachfolgend als Haltepunkte bezeichneten Stellen sind das Äquivalent tatsächlicher molekularer Wechselwirkungen zwischen Hautoberfläche und TTS. Ihre Anordnung erfolgt aus Gründen der Anschaulichkeit in regelmäßiger Verteilung über eine Fläche (Abb.2a und 2b), während die reale Verteilung als stochastisch anzunehmen ist.
Für die Haltepunkte wird weiterhin angenommen, daß jeder von ihnen mit seinen unmittelbar benachbarten Haltepunkten in direkter mechanischer Wechselwirkung steht.
Ein TTS nach dem Stand der Technik weist eine wesentlich geringere Elastizität auf als die menschliche Haut. In vielen Fällen kann ein TTS im Vergleich mit der Haut als Nahezu unelastisch gelten. Im Modell wird als Auswirkung von Scherung daher das Bestreben der Hautoberfläche dargestellt, gegen die vergleichsweise starre Schicht des aufgeklebten TTS eine Relativbewegung auszuführen. Diese Relativbewegung muß nicht die gesamte beklebte Fläche betreffen, sondern kann sich bei großen TTS auf Teile davon beschränken.
Für das Scherkraft-Modell gilt die realitätsnahe Prämisse einer sich vom TTS lösenden Haut gegenüber einer naheliegenden aber falschen Vorstellung eines sich von der Haut lösenden TTS.

Das Modell wird für die Betrachtung der Vorgänge an einem konvexen und einem konkaven Konturabschnitt herangezogen (Abb.2a bzw. 2b). Der sich in der konvexen Kontur ergebende Vorsprung der TTS-Fläche wird als Klippe bezeichnet, der vorderste Haltepunkt als Klippenpunkt (I) (Abb. 2a). In der konkaven Kontur erscheint dagegen eine Bucht und der am weitesten in dieser Bucht zurückliegende Haltepunkt wird als Buchtpunkt (II) bezeichnet (Abb.2b).

Die einwirkenden Scherkräfte werden durch Vektoren 2 dargestellt, die in der Ebenen der beklebten Haut 1 liegen. In der Vereinfachung richten sich diese entweder von der gezeigten TTS-Fläche 3 weg (offene Pfeilende) oder aber zu ihr hin (geschlossene Pfeilköpfe). Unter der Einwirkung dieser Scherkräfte kommt es zu mechanischen Wechselwirkungen der Haltepunkte untereinander. Diese Kräfte sind ebenfalls durch Vektoren 4 symbolisiert, deren Orientierung sich nach denen der einwirkenden Scherkräfte richtet und die daher in der Zeichnung die entsprechende Pfeilkopfdarstellung besitzen.
Währen der Klippenpunkt I in Abb. 2a mit nur drei benachbarten Haltepunkten B,C,D in Wechselwirkung tritt, steht der Buchtpunkt II in Abb. 2b in Wechselwirkung mit fünf seiner Nachbarn.
Dagegen ist die Anzahl von Wechselwirkungsmöglichkeiten für andere am Rand der Fläche liegende Haltepunkte B,D in Abb. 2a und C, D in Abb. 2b sowohl in einer Bucht als auch an einer Klippe identisch.

Die unterschiedlichen Verhältnisse an Klippenpunkt I und Buchtpunkt II führen zu einem bei gleicher Scherung bevorzugten Abriß der Haut 1 vom Klippenpunkt I gegenüber dem Buchtpunkt II. Dies beruht auf dem Umstand, daß der Halt der Haut 1 an einem Klippenpunkt I von weniger benachbarten Haltepunkten unterstützt wird als an einem Buchtpunkt II.

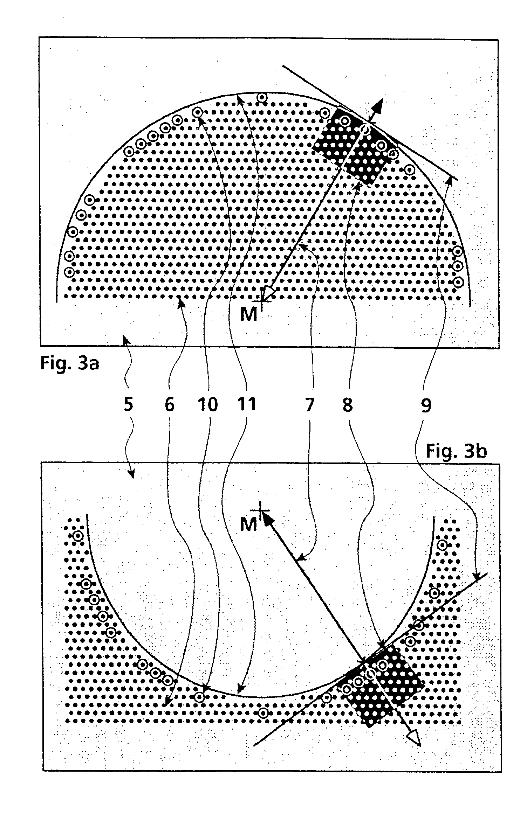

Würde in einem konkaven, buchtförmigen Konturabschnitt eines TTS nur ein einziger Buchtpunkt II, im entgegengesetzten Fall eines klippenförmigen konvexen Abschnittes nur ein einziger Klippenpunkt existieren, so wäre der daraus resultierende Vorteil vernachlässigbar. Tatsächlich existiert jedoch in einem gekrümmten Konturlinienabschnitt eine sehr große, endliche Zahl solcher Punkte in Abhängigkeit von der Richtung des Vektorfeldes der Scherkraft in der beklebten Hautfläche 1. In Abb. 3a und 3b wird dies an einer halbkreisförmigen Klippe bzw. einer halbkreisförmigen Bucht dargestellt. In Abhängigkeit von der Orientierung der Scherkraft 3 gemäß Abbildung 2a, 2b ergeben sich Klippen- bzw. Buchtpunkte an der Kante des TTS 2. Diese besonderen Haltepunkte 6 sind in den Abbildungen 3 a und 3 b eingekreist. Ihre Lage ergibt sich in der vereinfachten Darstellung nahe dem Schnittpunkt der TTS-Kontur 11 mit demjenigen Scherkraftvektor 7, der durch den Mittelpunkt M des Kreisbogens gelegt ist. Mit Hilfe der Tangente 9 am Kreisbogen 11 durch diesen Schnittpunkt läßt sich eine Vielzahl von Ausschnitten in Analogie zu den in Abbildung 2a und 2 b gezeigten identifizieren.
Aus der Vielzahl möglicher Bucht- bzw. Klippenpunkte innerhalb einer gekrümmten TTS-Kontur läßt sich ein deutlicher Vorteil von konkaven Konturlinien gegenüber konvexen erkennen.

Ein weiterer Vorteil der erfindungsgemäßen Kontur betrifft den Einfluß von Schälkräften auf die Ablösung eines TTS von der Haut 1.

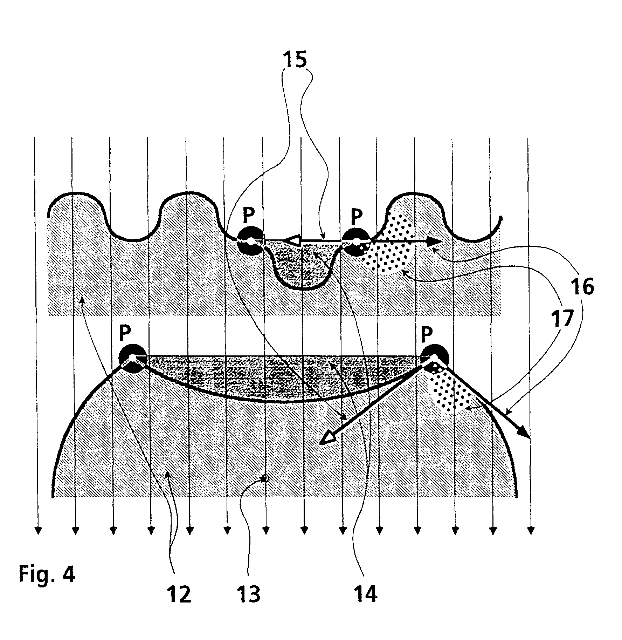

Solange ein konturschlüssiger Verbund des TTS mit der Haut besteht, haben Schälkräfte wenig Angriffsmöglichkeiten. Ist jedoch eine abgelöste Stelle vorhanden, so kann die Ablösung, insbesondere im Reibungskontakt mit Textilien, durch Schälung rasch fortschreiten. Die Klebrigkeit der abgelösten Schicht wird durch die anhaftenden Hautoberflächenbestandteile sowie durch hinzutretende Textilfasern so stark gemindert, daß eine erneute , dauerhafte Verklebung der abgelösten Partie mit der Haut praktisch nicht möglich ist. Eine Teilablösung des TTS während des Tragens ist daher in der Regel irreversibel, so daß Vorbeugemaßnahmen besondere Bedeutung besitzen. In Abbildung 4 ist der Ablösungsvorgang für eine wellenförmige sowie für eine einfache, bogenförmig konvexe Konturlinie schematisch dargestellt.
Es werden Ausschnitte einer auf der Haut klebenden TTS-Fläche 12 gezeigt, von der sich jeweils ein Teil 14 abgelöst hat. Die abgelösten Teile 14 werden vereinfacht zur Innenseite des TTS hin im Winkel von 180° abgekappt dargestellt. Die ablösend wirkenden Schälkräfte entsprechen einem Feld von TTS-einwärts gerichteten Vektoren. Dies kommt z.B. der Einwirkung einer der Haut in der Regel flach anliegenden Schicht von Textilien nahe, die mit den abgelösten TTS-Flächenteilen 14 verklebt und maßgeblich in einem Abzugswinkel von 180° ablösend wirkt.
Besondere Bedeutung für die theoretische Betrachtung besitzen diejenigen Punkte P, an denen die bereits abgelöste TTS-Kante mit der noch der Haut anhaftenden TTS-Kante zusammentrifft. Im Falle einer wellenförmigen Kontur verläuft die Ablösung zunächst weitgehend wie bei einem einfachen, konvexen Zuschnitt bis zu dem in Abbildung 4 gezeigten Zustand. Am Punkt P besitzt die abgelöste Konturlinie jetzt dieselbe räumliche Orientierung in der Ebene wie die noch anhaftende. Dies wird durch entsprechende Orientierungsvektoren 15, 16 dargestellt. Im Sinne einer mathematischen Kurvendiskussion besitzt die Konturlinie hier die Steigung Null.

Im 180°-Abzugswinkel an der entstandenen Lasche angreifende Zugkräfte wirken an einer wellenförmigen Kontur jetzt über den Punkt P auf eine Fläche von Haltepunkten des TTS, die ausschnittweise in Abbildung 3a, 3b dargestellt ist 7. Es wird ersichtlich, daß die noch der Haut anhaftende nächste Ausbuchtung des TTS maßgeblich Haltepunkte enthält, die einer weiteren Ablösung entgegenwirken. Bei einer einfachen konvexen Kontur tritt dieser Zustand dagegen zu keiner Zeit ein. Am Punkt P liegen die Orientierungen der abgelösten und der noch klebenden Kante niemals auf einer gemeinsamen Geraden. Der Punkt P stellt zu jeder Zeit einen Klippenpunkt dar, so daß eine weitere Ablösung gemäß obigen Ausführungen sogar noch begünstigt wird.

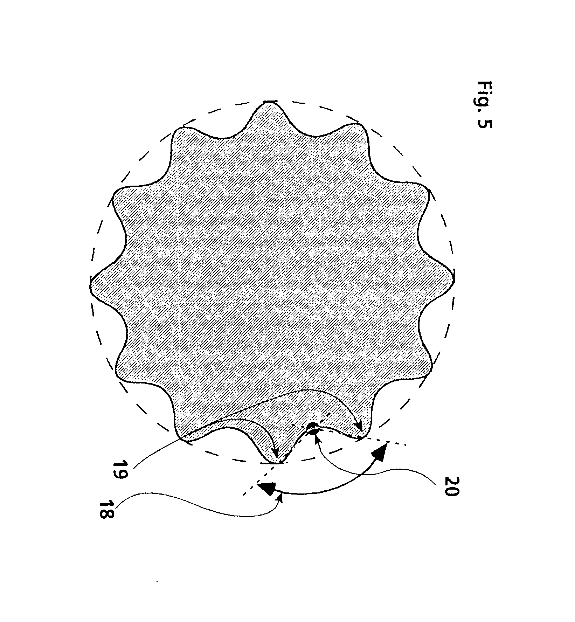

Ein weiterer Vorteil einer wellenförmigen Kontur liegt in der besseren Anpassung des TTS an gewölbte Applikationsflächen und betrifft insbesondere großflächige TTS. Unter gewölbten Applikationsflächen sind hier Flächen zu verstehen, die in mehr als einer Raumrichtung gekrümmt sind. Eine nur in einer Raumrichtung gekrümmte Fläche (Beispiel Zylinderoberfläche) kann faltenfrei mit einem TTS bedeckt werden, welches aus einem ebenen, flächigen Material ausgestanzt wurde. Für eine in mehreren Raumrichtungen gekrümmte Fläche (Beispiel: Oberfläche einer Halbkugel) gilt dies jedoch nicht. Während die Oberfläche einer Halbkugel mit der 3. Potenz ihres Radius zunimmt, vergrößert sich die Fläche eines kreisförmigen TTS nur mit der 2. Potenz des Radius. Daraus resultiert die Unmöglichkeit der faltenfreien Bedeckung einer Halbkugeloberfläche mit einem als ebene Fläche hergestellten TTS. Diese Problematik läßt sich auf das Bekleben bestimmter Hautareale des Körpers übertragen. So können z.B. Teile von Schulter, Brust, Bauch und Gesäß näherungsweise als Oberflächen von Halbkugeln betrachtet werden, und die Applikation großflächiger TTS führt hier leicht zu Faltenbildung. Durch Faltenbildung wird jedoch die Kontaktfläche zur Haut verkleinert und damit die Wirkstoffabgabe pro Zeit aus dem TTS unerwünscht verringert. Darüber hinaus wird die Ablösung des TTS von der Haut durch Haft- und Gleitreibung an hochstehenden Falten beschleunigt.
Prinzipiell kann die Problematik durch Einsatz elastischer oder plastischer Materialien bei der TTS- Herstellung gemildert werden. Solche Materialien wären in der Lage, das gegenüber der ebenen Fläche mit dem Radius überproportionale Wachstum einer Kugeloberfläche durch elastische oder plastische Dehnung mitzuvollziehen. Sowohl die plastische als auch die elastische Dehnung eines TTS gehen jedoch mit einer Vergrößerung seiner ursprünglichen Fläche bei gleichzeitiger Verringerung der Dicke einher. Dies ist unerwünscht, da sowohl die Vergrößerung der Abgabefläche an die Haut, als auch eine Verringerung der Schichtdicke des TTS die Wirkstoffabgabe an den Körper unkontrollierbar beeinflussen.
Durch eine wellenförmige Kontur können Dehnungen und Falten beim Bekleben einer gewölbten Fläche zwar nicht grundsätzlich vermieden, jedoch entscheidend vermindert werden. Die in Abb. 5 übersteigert dargestellten klippenförmigen Fortsätze der TTS-Fläche vermögen die genannten Effekte insbsondere im Randbereich aufzufangen. Durch das überproportionale Anwachsen der zu beklebenden, gewölbten Fläche verursachte Dehnungen können durch Spreizung 18 zweier benachbarter klippenförmiger Abschnitte 19 weitgehend kompensiert werden. Die plastische oder elastische Verformung des TTS reduziert sich dabei auf kleine Bereiche an den dazwischenliegenden Buchtpunkten 20. Insbesondere im Randbereich wird so der unter dem Gesichtspunkt der Langzeitklebung wichtige, konturschlüssige Verbund des TTS mit der Haut erleichtert.

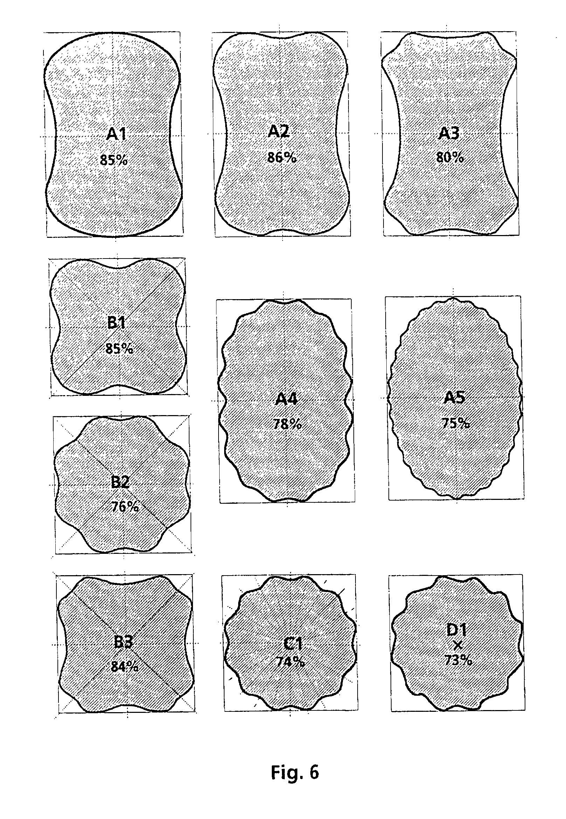

Zusammenfassend weisen solche TTS, die erfindungsgemäß konkave Abschnitte in ihrer Kontur enthalten, eine Reihe von Vorteilen betreffend den langfristigen konturschlüssigen Verbund mit der Haut auf. Sie sind dabei meist ohne technischen Mehraufwand gegenüber den üblichen TTS-Herstellungsweisen zu fertigen und weisen eine Flächenausnutzung auf, die nur unwesentlich geringer ist als bei den nach dem Stand der Technik gebräuchlichen TTS-Konturen.
Eine beispielhafte, aber nicht vollständige Übersicht über die erfindungsgemäß neuartigen Konturen und ihre bevorzugte Ausprägung gibt Figur 6.
Systeme vom Typ A sind durch das Vorhandensein von 2 Symmetrieebenen in der Kontur gekennzeichnet. Dargestellt sind bevorzugte Varianten dieses Typs mit 2 (A¹), 4 (A²) und 8 (A³) konkaven Abschnitten in der Kontur. Bei einer noch größeren Anzahl von konkaven Abschnitten ergeben sich beispielsweise Konturen mit einem grob (A⁴) oder fein (A⁵) wellenförmigen Verlauf. Die Systeme vom Typ A weisen in der Kontur eine gerade Anzahl von n=2 bis n=50 konkaven Abschnitten auf.
Systeme vom Typ B sind durch das Vorhandensein von 4 Symmetrieebenen gekennzeichnet. Die bevorzugten Varianten weisen in der Kontur 4 (B¹) oder 8 (B²,B³) konkave Abschnitte auf. Auch eine größere Anzahl von konkaven Abschnitten ist möglich, aus der sich dann Konturen in Analogie zu denjenigen mit 2 Symmetrieebenen vom Typ A⁴ und A⁵ ergeben. Die Systeme vom Typ B weisen in der Kontur eine gerade Anzahl von n=4 bis n=50 konkaven Abschnitten auf. Systeme vom Typ C sind radiärsymmetrisch aufgebaut. Unter radiärsymmetrischem Verlauf ist zu verstehen, daß alle Symmetrieebenen an einem einzigen gemeinsamen Punkt innerhalb der Fläche des TTS beginnen. Die Anzahl der konkaven Abschnitte in der Kontur beträgt n=5 bis n=50 und ist geradzahlig oder vorzugsweise ungeradzahlig.
Systeme vom Typ D weisen eine punktsymmetrisch verlaufende Kontur auf. Der Symmetriepunkt liegt innerhalb der Fläche des TTS. Die Anzahl der konkaven Abschnitte beträgt n=3 bis n=50 und die Zahl n nimmt geradzahlige oder ungeradzahlige Werte an.

In Abbildung 6 ist für jeden Ausbildungstyp (A¹,A²,A³,A⁴,A⁵,B¹,B²,B³,C¹,D¹) der Flächennutzungskoeffizient in Prozenten angegeben. Unter dem Flächennutzungskoeffizienten ist das Verhältnis der Fläche des TTS zu der Fläche des kleinsten seine Kontur umschreibenden Rechteckes zu verstehen. Höhere Werte bedeuten eine höhere Ausbeute bei der Herstellung von Einzelformen aus einem flächigen Vorprodukt und sind daher günstiger als niedrige.

## Patentansprüche

1. Transdermales therapeutisches System (TTS) zur Abgabe von Wirkstoffen an oder durch die Haut mit einer auf der Haut vergleichsweise dauerhaft haftklebenden Schicht, deren äußere Ränder durch eine Kontur bestimmt sind, **dadurch gekennzeichnet, daß** in der Kontur wenigstens zwei relativ zur Fläche konkave Abschnitte enthalten sind.

2. TTS nach Anspruch 1, **dadurch gekennzeichnet, daß** auf die konkaven Konturabschnitte in der Summe mindestens 30 % der Gesamtlänge der Kontur entfallen.

3. TTS nach Anspruch 1 und 2, **dadurch gekennzeichnet, daß** die Kontur 2 Symmetrieebenen (x-x;y-y) aufweist und die Anzahl der konkaven Abschnitte n=2 bis n=50 beträgt und geradzahlig ist.

4. TTS nach Anspruch 1 uns 2, **dadurch gekennzeichnet, daß** die Kontur 4 Symmetrieebenen (x-x; y-y; v-v; w-w) aufweist und die Anzahl der konkaven Abschnitte n=4 bis n=50 beträgt und geradzahlig ist.

5. TTS nach Anspruch 1 und 2, **dadurch gekennzeichnet, daß** die Kontur radiärsymmetrisch verläuft und n=3 bis n=50 konkave Abschnitte enthält, wobei die Zahl n geradzahlige oder ungeradzahlige Werte annimmt.

6. TTS nach Anspruch 1 und 2, **dadurch gekennzeichnet, daß** die Kontur punktsymmetrisch zu einem in der Fläche des TTs liegenden Punkt verläuft und n=3 bis n=50 konkave Abschnitte enthält, wobei die Zahl n geradzahlige oder ungeradzahlige Werte annimmt.

## Claims

1. Transdermal therapeutic system (TTS) for the delivery of active compounds to or through the skin, having a layer adhering comparatively permanently to the skin, whose outer edges are defined by a contour, **characterized in that** at least two sections which are concave relative to the surface are contained in the contour.

2. TTS according to Claim 1, **characterized in that** at least 30% of the total length of the contour is allotted to the concave contour sections in total.

3. TTS according to Claims 1 and 2, **characterized in that** the contour has 2 planes of symmetry (x-x;y-y) and the number of concave sections is n=2 to n=50 and is even-numbered.

4. TTS according to Claims 1 and 2, **characterized in that** the contour has 4 planes of symmetry (x-x;y-y;v-v;w-w) and the number of concave sections is n=4 to n=50 and is even-numbered.

5. TTS according to Claims 1 and 2, **characterized in that** the contour runs radially symmetrically and contains n=3 to n=50 concave sections, the number n assuming even-numbered or odd-numbered values.

6. TTS according to Claims 1 and 2, **characterized in that** the contour runs point-symmetrically to a point lying in the surface of the TTS and contains n=3 to n=50 concave sections, the number n assuming even-numbered or odd-numbered values.

## Revendications

1. Système thérapeutique transdermique (STT) servant à délivrer des substances actives sur la peau ou à travers la peau avec une couche adhérant de manière relativement durable sur la peau et dont les bords extérieurs sont déterminés par un contour, **caractérisé en ce qu'**au moins deux segments concaves par rapport à la surface sont contenus dans le contour.

2. STT selon la revendication 1, **caractérisé en ce que** les segments concaves du contour représentent, au total, au moins 30% de la longueur totale du contour.

3. STT selon les revendications 1 et 2, **caractérisé en ce que** le contour a 2 plans de symétrie (x-x ; y-y) et le nombre de segments concaves va de n = 2 à n = 50 et est un nombre pair.

4. STT selon les revendications 1 et 2, **caractérisé en ce que** le contour a 4 plans de symétrie (x-x ; y-y ; v-v ; w-w) et le nombre de segments concaves va de n = 4 à n = 50 et est un nombre pair.

5. STT selon les revendications 1 et 2, **caractérisé en ce que** le contour présente une symétrie radiale et contient de n = 3 à n = 50 segments concaves, le nombre n prenant des valeurs paires ou impaires.

6. STT selon les revendications 1 et 2, **caractérisé en ce que** le contour présente une symétrie ponctuelle par rapport à un point situé dans la surface du STT et contient de n = 3 à n = 50 segments concaves, le nombre n prenant des valeurs paires ou impaires.
